(19) Europäisches Patentamt
European Patent Office
Office européen des brevets

(11) Veröffentlichungsnummer: **0 033 477**
**B1**

(12) # EUROPÄISCHE PATENTSCHRIFT

(45) Veröffentlichungstag der Patentschrift: **18.07.84**

(51) Int. Cl.³: **C 07 D 233/76**, C 08 G 18/78, C 08 G 73/06, C 09 D 3/49

(21) Anmeldenummer: **81100407.6**

(22) Anmeldetag: **21.01.81**

(54) **Verfahren zur Herstellung von Hydantoinen und deren Verwendung in temperaturbeständigen Lacken, Folien, Klebern, Schaumstoffen oder Formkörpern.**

(30) Priorität: **02.02.80 DE 3003773**

(43) Veröffentlichungstag der Anmeldung: **12.08.81 Patentblatt 81/32**

(45) Bekanntmachung des Hinweises auf die Patenterteilung: **18.07.84 Patentblatt 84/29**

(84) Benannte Vertragsstaaten: **AT CH DE FR GB IT LI NL**

(56) Entgegenhaltungen:
**EP - A - 0 002 444**
**EP - A - 0 002 445**
**EP - A - 0 002 662**
**DE - A - 2 539 730**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Zecher, Wilfried, Dr., Treptower Strasse 6, D-5090 Leverkusen (DE)**
Erfinder: **Merten, Rudolf, Dr., Berta-von-Suttner-Strasse 55, D-5090 Leverkusen (DE)**

EP 0 033 477 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es ist bekannt, daß man Hydantoine durch Umsetzung von $\alpha$-Amino-carbonsäurederivaten mit Isocyanaten erhält (Anm. Chem. J. 45, 383). Weitere Herstellungswege sind die Reaktion von $\alpha$-Aminonitrilen mit Isocyanaten und nachfolgende Verseifung der entstehenden Amino-Verbindungen und die Cyclisierung von $\alpha,\beta$-ungesättigten Dicarbonsäure-diamiden mit Isocyanaten (DOS-2 714 655). Monomolekulare Hydantoine können im Pharmabereich und auf dem Pflanzenschutzsektor eingesetzt werden, während Polyhydantoine als temperaturbeständige Kunststoffe, insbesondere auf dem Elektroisoliersektor, in die Technik Eingang gefunden haben (franz. Pat. 1 484 694).

Es wurde nun gefunden, daß man Hydantoine mit guten Ausbeuten erhält, wenn man organische Isocyanate mit gegebenenfalls substituierten Fumarsäuremonoestern der allgemeinen Formel

$$R^1{-}OOC{-}\overset{\overset{\displaystyle R_2}{|}}{C}{=}\overset{\overset{\displaystyle R_3}{|}}{C}{-}COOH$$

in der

$R^1$ einen aliphatischen Rest mit 1—22 C-Atomen, einen aliphatisch-aromatischen Rest mit 7—20 C-Atomen, einen aromatischen Rest mit 6—16 C-Atomen oder einen heterocyclischen Rest mit 3—10 Ring-C-Atomen und 1—3 N-, S- und/oder O-Atomen im Ring bedeutet,
$R^2$ und $R^3$ für Wasserstoff oder Methyl steht,

bei Temperaturen vor 0—450° C, vorzugsweise von 30 bis 250° C, umsetzt.

Der Reaktionsverlauf ist überraschend, da üblicherweise aus Carbonsäuren und Isocyanaten überwiegend die entsprechenden Säureanhydride und Harnstoffe gebildet werden und die cyclischen Carbonsäureanhydride, die aus den erfindungsgemäßen Carbonsäureestern durch Abspaltung des Alkohols gebildet werden können, mit Isocyanaten zu komplizierten, teilweise polymeren Stoffgemischen reagieren. Die von der Konstitution her vergleichbaren Maleinsäuremonoester ergeben dementsprechend bei der Umsetzung mit Isocyanaten Substanzgemische, die nur geringe Anteile von Hydantoinen enthalten.

Auch die relativ guten Ausbeuten an Hydantoinen, die sogar den Aufbau von Polymeren ermöglichen, waren bei der erfindungsgemäßen Reaktion, die über mehrere Stufen verläuft, nicht zu erwarten. Außerdem wird bei der Umsetzung nur $CO_2$ frei und nicht, wie bei bekannten Verfahren, Wasser oder Alkohole, die zu störenden Nebenreaktionen führen können.

Die erfindungsgemäß als Ausgangsmaterialien verwendeten Fumarsäuremonoester sind z. B. aus der Fumarsäure oder ihren Substitutionsprodukten durch partielle Veresterung oder aus Maleinsäureestern durch Umlagerung zugänglich und können in Substanz eingesetzt oder auch im Reaktionsmedium aus den Komponenten hergestellt werden.

Der Rest $R_1$ leitet sich vorzugsweise von Methan, Ethan, n-, iso-, tert.-Butan, Hexan, Eicosan, Propen, Butin, Cyclohexan, Benzol, Naphthalin, Diphenylmethan, Diphenylether, Diphenylsulfon, $\omega$- oder kernsubstituierten Toluol, Xylol, Polyethern, Polyestern, Polyharnstoffen und Polyurethanen ab und kann einfach oder mehrfach, z. B. mit Halogen oder Alkyl-$C_1$—$C_6$ oder Aryl-Resten-$C_6$—$C_{10}$, Carbonsäure- oder Carbonsäureestergruppen substituiert sein.

Die bevorzugten Verbindungen erhält man z. B. durch Umsetzung der Fumarsäure zu den Monoestern mit Methanol, Ethanol, Butanol, Isopropylalkohol, Phenol und Kresol.

Besonders bevorzugt werden die $C_1$—$C_6$-Monoalkylester der Fumarsäure eingesetzt.

Für das erfindungsgemäße Verfahren eignen sich Mono- bzw. Polyisocyanate der allgemeinen Formel

$$R_4(—NCO)_z$$

in welcher $R_4$ für einen, gegebenenfalls mit Halogen, Alkyl- und/oder Arylgruppen substituierten aliphatischen Rest mit 1—20 C-Atomen, einen aromatischen Rest mit 6—12 C-Atomen, einen cycloaliphatischen Rest mit 5—12 C-Atomen und einen aliphatischen-aromatischen Rest mit 7—20 C-Atomen steht. Besonders bevorzugt sind Alkyl-Reste mit 2—12 C-Atomen, oder ein Arylrest wie Phenyl, Tolyl, Naphthyl, Diphenylmethan und Diphenyletherreste. z ist eine ganze Zahl von 1—4, vorzugsweise 1—3, besonders bevorzugt 2.

Monoisocyanate im Sinne der Erfindung sind z. B. Alkylisocyanate wie Ethyl-, Methyl-, Butyl-, Dodecyl- und Stearylisocyanat, aromatische, gegebenenfalls substituierte Monoisocyanate wie Phenyl-, Tolyl-, Isopropyl-, Nonylisocyanat, Nitro-, Alkoxy-, Aroxy-, Chlor-, Dichlor-, Trichlor-, Tetra-, Pentachlor-, Benzyl-, Brom-phenyl-isocyanat oder Isocyanatobenzoesäureester, Phthalsäureester, Isophthalsäureester, Isocyanatobenzonitril, cycloaliphatische Isocyanate wie Cyclohexylisocyanat und ungesättigte Isocyanate wie Allyl-, Oleyl-; Cyclohexenyl-isocyanat.

Erfindungsgemäß einzusetzende Polyisocyanate, vorzugsweise Diisocyanate (vgl. Annalen, 562,

Seite 75 bis 136) sind beispielsweise Ethylendiisocyanat, 1,4-Tetramethylendiisocyanat, 1,6-Hexamethylen-diisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanato-methyl-cyclohexan (DAS 1 202 785), 2,4- und 2,6-Hexahydrotoluylendiisocyanat sowie beliebige Gemische dieser Isomeren, Hexahydro-1,3- und/oder -1,4-phenylen-diisocyanat, Perhydro-2,4′ und/oder -4,4′-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4′- und/oder 4,4′-diisocyanat, Naphthylen-1,5-diisocyanat, Triphenylmethan-4,4′,4″-Triisocyanat, Polyphenyl-polymethylen-polyisocyanate, wie sie durch Anilin-Formaldehyd-Kondensation und anschließende Phosgenierung erhalten und z. B. in den britischen Patentschriften 874 430 und 848 671 beschrieben werden, perchlorierte Arylpolyisocyanate, wie sie z. B. in der deutschen Auslegeschrift 1 157 601 beschrieben werden, Carbodiimidgruppen aufweisende Polyisocyanate, wie sie in der deutschen Patentschrift 1 092 007 beschrieben werden, Diisocyanate, wie sie in der US-Patentschrift 3 492 330 beschrieben werden, Allophanatgruppen aufweisende Polyisocyanate, wie sie z. B. in der britischen Patentschrift 99 890, der belgischen 761 626 und der veröffentlichten holländischen Patentanmeldung 7 102 524 beschrieben werden, Isocyanuratgruppen aufweisende Polyisocyanate, wie sie z. B. in den deutschen Patentschriften 1 022 789, 1 222 067 und 1 027 394 sowie in den deutschen Offenlegungsschriften 1 929 034 und 2 004 048 beschrieben werden, Urethangruppen aufweisende Polyisocyanate, wie sie z. B. in der belgischen Patentschrift 752 261 oder in der US-Patentschrift 3 394 164 beschrieben werden, acylierte Harnstoffgruppen aufweisende Polyisocyanate gemäß der deutschen Patentschrift 1 230 778, Biuretgruppen aufweisende Polyisocyanate, wie sie z. B. in der deutschen Patentschrift 1 101 394 in der britischen Patentschrift 889 050 und in der französischen Patentschrift 7 017 514 beschrieben werden, durch Telomerisationsreaktionen hergestellte Polyisocyanate, wie sie z. B. in der belgischen Patentschrift 723 640 beschrieben werden, Estergruppen aufweisende Polyisocyanate, wie sie z. B. in den britischen Patentschriften 956 474 und 1 072 956, in der US-Patentschrift 3 567 763 und in der deutschen Patentschrift 1 231 688 genannt werden, Umsetzungsprodukte der obengenannten Isocyanate mit Acetylen gemäß der deutschen Patentschrift 1 072 358.

Es ist auch möglich, die bei der technischen Isocyanatherstellung anfallenden Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Bevorzugt verwendet man die technisch leicht zugänglichen Gemische aus Toluylen-diisocyanaten, m-Phenylendiisocyanat, Phenylisocyanat und seine Substitutionsprodukte, Methylisocyanat, sowie phosgenierte Kondensate aus Anilin und Formaldehyd mit Polyphenylenmethylen-struktur und die symmetrischen Verbindungen 4,4′-Diisocyanato-diphenylmethan, 4,4′-Diisocyanato-diphenylether, p-Phenylendiisocyanat, 4,4′-Diisocyanato-diphenyl-dimethylmethan, analoge hydroaromatische Diisocyanate, sowie aliphatische Diisocyanate mit 2—12 C-Atomen wie Hexamethylendiisocyanat und von Isophoron abgeleitete Diisocyanate.

Die Isocyanate können in freier Form, ferner zum Teil oder vollständig auch in Form ihrer, beim Umsatz mit reaktiven Wasserstoff enthaltenden Verbindungen, zugänglichen und unter den Reaktionsbedingungen als Abspalter reagierenden Derivate eingesetzt werden.

Vorzugsweise werden als Abspalter die aus Lactamen, z. B. Caprolactam, zugänglichen Acyl-Harnstoffe und die aus aromatischen und aliphatischen Mono- und Polyhydroxy-Verbindungen erhaltenen Carbamidsäureester, die z. B. den allgemeinen Formeln

$$\left(R_4-NH-\underset{\underset{O}{\|}}{C}-O-A\right)_z$$

bzw.

$$\left[-\underset{\underset{O}{\|}}{C}-NH-R_4-NH-\underset{\underset{O}{\|}}{C}-O-B-O-\right]_n$$

entsprechen, eingesetzt, wobei $R_4$ und z die oben angegebene Bedeutung haben und A der organische Rest einer Monohydroxyverbindung bzw. B der organische Rest einer bis- oder trisfunktionellen Hydroxyverbindung, vorzugsweise ein aliphatischer Rest mit 1—10 C-Atomen, einen cycloaliphatischen Rest mit 5—10 C-Atomen, ein aliphatischer-aromatischer Rest mit 7—12 C-Atomen und ein aromatischer Rest mit 6—12 C-Atomen ist, die jeweils auch mit Alkyl- und/oder Arylgruppen substituiert sein können, und n für eine ganze Zahl von 1—1000, vorzugsweise 1—100 steht.

Als Beispiele seien die Carbamidsäureester aus Phenol, isomeren Kresolen, deren technischen Gemischen und ähnlichen aromatischen Hydroxylverbindungen, aliphatische Monoalkohole wie Methanol, Ethanol, Propanol, Isopropanol, Butanol, Isobutanol, Diethylenglykolmonomethylether, Cyclohexanol, Benzylalkohol und aliphatische Di- oder Polyole wie Ethylenglykol und Trimethylolpropan aufgeführt.

Die Urethane können als solche eingesetzt oder erst in situ durch Umsetzung mit Alkoholen erzeugt werden.

Durch das nachfolgende Reaktionsschema soll die erfindungsgemäße Reaktion erläutert werden:

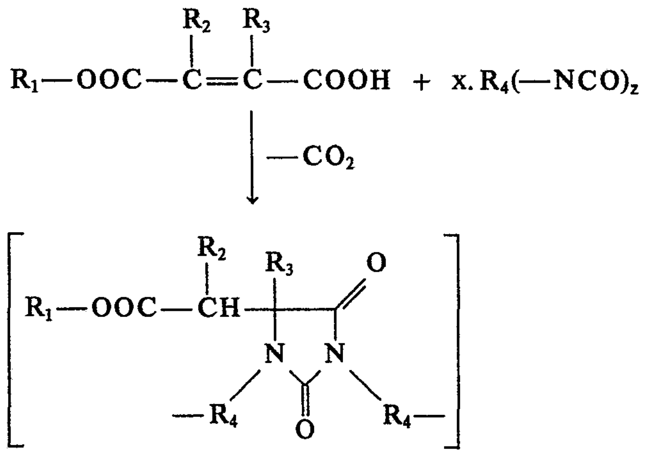

wobei die Reste $R_1$, $R_2$, $R_3$ und $R_4$ die oben angegebene Bedeutung haben, $x=1$ oder 2 ist und für $z=1$ eine monomolekulare und für $z>1$ eine höhermolekulare Verbindung entsteht, wobei die Hydantoinringe über die Reste $R_4$— verknüpft sind.

Die erfindungsgemäßen Hydantoinester können eindeutig durch IR-Spektren, in denen die charakteristischen Banden für Hydantoine und Ester auftreten, identifiziert werden. Die höhermolekularen Hydantoine zeigen eine Lösungsviskosität von 200 bis 200 000 mPa · s, vorzugsweise von 1000 bis 50 000, die an einer 30gew.-%igen Lösung in Butyrolaoton bei 25° C bestimmt wird.

Die erfindungsgemäße Reaktion kann in Lösungsmitteln, die unter den Reaktionsbedingungen nicht reagieren oder nur lockere Additionsverbindungen bilden, oder auch in einem Überschuß einer der Reaktionskomponenten ausgeführt werden. Geeignete Lösungsmittel sind: (Halogen)-Kohlenwasserstoffe, Phenole, Alkohole, Ester, Lactone, Ketone, Ether, substituierte Amide, Nitrile, Phosphorsäureamide, Sulfoxide und Sulfone, beispielsweise Xylole, o-Dichlorbenzol, Phenol, Kresole, Benzoesäurealkylester, Butyrolacton, Caprolacton, Acetonphenon, Cyclohexanon, Benzylalkohol, Ethylenglykol, Glykolmonomethyletheracetat, Diethylenglykolmonoethylether, Diethylenglykoldimethylether, Dimethylformamid, N-Methylpyrrolidon, Caprolactam, Benzonitril, Hexamethylphosphorsäuretriamid, Dimethylsulfoxid, Tetramethylensulfon und deren Gemische.

Die Durchführung des erfindungsgemäßen Verfahrens erfolgt in der Weise, daß die Reaktionskomponenten mit oder ohne Lösungsmittel einige Minuten bis zu mehreren Stunden bei Temperaturen von etwa 0—450° C, vorzugsweise 30—250° C gehalten werden. Der Verlauf der Reaktion läßt sich über die Gasentwicklung und die IR-Spektren verfolgen. Es ist zuweilen vorteilhaft, die Reaktion in mehreren Stufen durchzuführen oder die einzelnen Komponenten in unterschiedlicher Reihenfolge oder bei verschiedenen Temperaturen zuzugeben. Insbesondere bei der Herstellung von Polymeren kann auch in einer ersten Stufe, z. B. in einem Lösungsmittel, ein Kondensationsprodukt hergestellt werden, das dann bei höheren Temperaturen unter Kettenverlängerung oder Vernetzung und gegebenenfalls Verdampfen des Lösungsmittels, in das hochmolekulare Reaktionsprodukt, z. B. einen Lackfilm, übergeführt wird. Bei der Verwendung in Lacken können diese auch aus Schmelzen oder wäßrigen Dispersionen appliziert werden.

Im allgemeinen werden pro Val Monoester 2 Val Isocyanat eingesetzt, doch sind auch sehr weitgehende Abweichungen von diesen Mengenverhältnissen möglich. Aus Monoestern und monofunktionellen Isocyanaten werden monomolekulare Hydantoine, aus Monoestern und Diisocyanaten in Abhängigkeit von den stöchiometrischen Verhältnissen höhermolekulare Polyhydantoine oder Oligomere, z. B. Hydantoinisocyanate, erhalten.

Die erfindungsgemäße Reaktion kann durch Katalysatoren beeinflußt werden, z. B. durch Amine wie Triethylamin, 1,4-Diazabicyclo-(2,2,2)octan, N-Ethyl-morpholin und N-Methyl-imidazol sowie durch organische und anorganische Metallverbindungen, insbesondere von Eisen, Blei, Zink, Zinn, Kupfer, Kobalt und Titan, wie Eisen(III)-chlorid, Kobaltacetat, Bleioxid, Bleiacetat, Zinnoctoat, Dibutylzinn-dilaurat, Kupfer-acetyl-acetonat, Titantetrabutylat, Alkali-phenolate und Natriumcyanid und durch Phosphorverbindungen wie Trialkylphosphin und Methylphospholinoxid und Hydroxy-Aromaten wie Phenol, Hydrochinon und Resorcin.

Die nach dem erfindungsgemäßen Verfahren herstellbaren monomolekularen Hydantoine zeigen Wirkungen auf dem pharmazeutischen und dem Pflanzenschutz-Sektor. Die erfindungsgemäßen Polyhydantoine zeichnen sich durch besondere Temperaturbeständigkeit aus und sind geeignet zur

Verwendung als Kleber, Lacke, Folien und Formkörper. Ihre Eigenschaften können für die verschiedenen Einsatzgebiete durch Zusatz von Füllstoffen, Pigmenten und nieder- und hochmolekularen Komponenten, z. B. zur Herstellung von Lacken und Folien durch Abmischen mit Polyestern und Polycarbamidestern, in weiten Grenzen variiert werden, wie die z. B. in DOS-2 654 112 beschrieben wird.

Beispiel 1

79 g Fumarsäuremonoisopropylester und 153,5 g 4-Chlorphenylisocyanat werden in 230 g N-Methylpyrrolidon eingetragen. Dann wird jeweils 2 Stunden bei 80°, 100°, 120° und 130°C gerührt. Die Kondensation und Cyclisierung zum Hydantoin erfolgt unter Abspaltung von Kohlendioxid. Das Reaktionsgemisch wird filtriert und unter Rühren mit 100 ml Wasser versetzt. Man erhält 156 g 1,3-Bis-(4-chlorphenyl)-hydantoyl-(5)-essigsäureisopropylester in farblosen Polyedern vom Schmp. 136—138°C. Nach Umkristallisation aus Acetonitril beträgt der Schmp. 142—143°C. Das IR-Spektrum zeigt bei 1725 und 1785 $Cn^{-M}$ die für Hydantoine und Ester charakteristischen Banden.

$C_{20}H_{18}Cl_2N_2O_4$ (420,0)

| | | | |
|---|---|---|---|
| ber.: | C 57,0, | H 4,3, | N 6,7%; |
| gef.: | C 57,2, | H 4,3, | N 7,0%. |

Beispiel 2

In 185 g Dimethylformamid werden 65 g Fumarsäuremonomethylester und 119 g Phenylisocyanat unter Rühren in jeweils 2 Stunden bei 80°, 100°, 120° und 130°C zur Umsetzung gebracht. Die Lösung des Reaktionsprodukts wird in Wasser eingerührt und kristallisiert nach einiger Zeit zu 151 g einer braunen Masse, die in Chloroform aufgenommen, filtriert und eingedampft wird. Die Umkristallisation des Rückstands aus Methanol ergibt den 1,3-Diphenyl-hydantoyl-(5)-essigsäuremethylester in farblosen Polyedern vom Schmp. 146—148°C und den für Hydantoine und Ester-Gruppen im IR-Spektrum charakteristischen Banden bei 1710, 1720 und 1765 $cm^{-1}$.

$C_{18}H_{16}N_2O_4$ (324)

| | | | |
|---|---|---|---|
| ber.: | C 66,7, | H 4,9, | N 8,6%; |
| gef.: | C 66,6, | H 5,2, | N 8,8%. |

Beispiel 3

28,8 g Fumarsäuremonoethylester und 47,6 g Phenylisocyanat werden in 55 g N-Methylpyrrolidon eingetragen und unter Rühren auf 120°C erhitzt. Dann wird die Temperatur im Verlaufe von 4 Stunden auf 160°C gesteigert und noch 2 Stunden bei diesem Wert gehalten. Nach dem Erkalten wird das Reaktionsgemisch in Wasser eingerührt. Man erhält eine feste Ausscheidung, die, aus Methanol umkristallisiert, 57 g 1,3-Diphenyl-hydantoyl-(5)-essigsäureethylester vom Schmp. 74—78°C ergibt. Der Schmp. der reinen Verbindung beträgt nach Umkristallisation aus Isopropanol und Ethanol 84—85°C.

$C_{19}H_{18}N_2O_4$ (338)

| | | | |
|---|---|---|---|
| ber.: | C 67,5, | H 5,3, | N 8,3%; |
| gef.: | C 67,6, | H 5,7, | N 8,5%. |

Beispiel 4

72 g Fumarsäuremonoethylester und 125 g 4,4'-Diisocyanato-diphenylmethan werden in 350 g N-Methylpyrrolidon gelöst. Dann wird auf 80°, 100°, 120°, 130° und 140°C erhitzt. Im Verlaufe der Kondensation und Cyclisierung werden ca. 13 l Kohlendioxid abgespalten. Man erhält den Polyhydantoinethylester als klare braune Lösung mit einer Viskosität $\eta$ 25=420 mPa · s und Hydantoin- und Ester-Banden bei 1725 und 1780 $m^{-1}$. Eine Probe der Polyhydantoin-Lösung wird auf ein Prüfblech aufgestrichen und in jeweils 15 Minuten bei 200° und 300°C zu einem klaren elastischen Lackfilm eingebrannt.

## Beispiel 5

72 g Fumarsäuremonoethylester, 125 g 4,4'-Diisocyanato-diphenylmethan und 1 g Diaza-bicyclo-octan werden in 275 g eines technischen Kresolgemisches eingetragen. Dann wird 4 Stunden bei 170° und 4 Stunden bei 190° C gerührt, 175 g Kresol zugegeben und die Reaktion in 6 Stunden bei 190° C zu Ende gerührt. Der Polyhydantoinethylester wird als viskose Lösung mit Hydantoin- und Ester-Banden bei 1710 und 1770 $cm^{-1}$ erhalten. Eine Probe der Hydantoin-Lösung wird mit einem Polyester aus Terephthalsäure, Ethylenglykol und Glycerin im Verhältnis 1 : 1, bezogen auf den Festgehalt, abgemischt, mit Kresol verdünnt und auf eine Glasplatte aufgestrichen. Man erhält nach dem Einbrennen in jeweils 15 Min. bei 200° und 300° eine klare elastische Folie.

## Beispiel 6

In 548 g einer Mischung aus gleichen Teilen Phenol und einem technischen Kresol-Gemisch werden bei 120° C 144 g Fumarsäuremonoethylester und 174 g einer Mischung aus 80% 2,4- und 20% 2,6-Toluylendiisocyanat eingetragen. Dann wird auf 160° C aufgeheizt und 2 Stunden bei 160° C, 4 Stunden bei 180°, 10 Stunden bei 190° und 4 Stunden bei 200° gerührt. Man erhält den Polyhydantoinethylester als klare braune Lösung mit einer Viskosität $\eta 25 = 1320$ mPa · s und einem Festgehalt von ca. 33%. Das IR-Spektrum enthält bei 1714 und 1775 $cm^{-1}$ die für Hydantoine und Ester charakteristischen Banden. Eine Probe der Lösung wird mit, bezogen auf den Festgehalt, gleichen Teilen eines Polyesters aus Terephthalsäure, Ethylenglykol und Glycerin und 1,5% Titantetrabutylat versetzt, auf eine Glasplatte aufgestrichen und in jeweils 15 Minuten bei 200 und 300° C zu einem klaren harten Lackfilm eingebrannt.

## Beispiel 7

90 g eines technischen Gemisches aus 80% 2,4- und 20% 2,6-Toluylendiisocyanat, 79 g Fumarsäureisopropylester und 0,9 g Diaza-bicyclooctan werden in 340 g Butyrolacton unter Rühren jeweils in 2 Stunden bei 80, 100 und 120° C und 4 Stunden bei 140° C zum Polyhydantoin-isopropylester unter Abspaltung von 14 l Kohlendioxid kondensiert. Die Viskosität $\eta$ 25 der so hergestellten braunen Hydantoin-Lösung beträgt 2490 mPa · s, die Hydantoin- und Ester-Banden im IR-Spektrum liegen bei 1730 und 1775 $cm^{-1}$. In jeweils 15 Minuten bei 200 und 300° C wird aus der Lösung auf einem Prüfblech ein klarer harter Lackfilm erhalten.

## Beispiel 8

In 340 g Solvesso 100, einem technischen Gemisch aus Alkylaromaten, werden 130 g Fumarsäuremonomethylester, 250 g 4,4'-Diisocyanato-diphenylmethan und 1,2 g Diazabicyclo-octan eingetragen. Dann wird 4 Stunden auf 80°, 4 Stunden auf 100°, 2 Stunden auf 120° und 2 Stunden auf 130° C erhitzt. Das Reaktionsprodukt, eine heterogene braune Masse, wird abgesaugt und der Filterrückstand im Vakuum getrocknet. Man erhält den Polyhydantoin-methylester als hellbraunes Pulver mit den Hydantoinen und Estern entsprechenden Banden im IR-Spektrum bei 1725 und 1780 $cm^{-1}$.

$(C_{19}H_{16}N_2O_4)_n$ (336)n
N: ber.: 8,3%;
gef.: 8,1%.

Eine Probe des Polyhydantoins wird bei 300° C unter Stickstoff zu einem harten Formkörper gesintert. Eine weitere Probe wird in Kresol gelöst und bei 200 und 300° C zu einem klaren elastischen Lackfilm eingebrannt.

## Beispiel 9

In 288 g Dimethylacetamid werden 79 g Fumarsäuremonoisopropylester, 87 g Hexamethylendiisocyanat und 0,9 g Diaza-bicyclo-octan als Katalysator eingetragen. Dann wird jeweils 2 Stunden bei 80, 100, 120 und 130° C, 10 Stunden bei 150 und 2 Stunden bei 170° C gerührt. Man erhält den aliphatischen Polyhydantoinisopropylester als braune 33%ige Lösung mit der Viskosität $\eta 25 = 50$ mPa · s und den für Hydantoine und Ester charakteristischen Banden im IR-Spektrum bei 1715 und 1770 $cm^{-1}$. Die Hydantoin-Lösung wird auf eine Glasplatte aufgestrichen und in jeweils 15 Minuten bei 200 und 300° C zu einem klaren elastischen Lackfilm eingebrannt.

**Patentansprüche**

1. Verfahren zur Herstellung von Hydantoinen der Formel

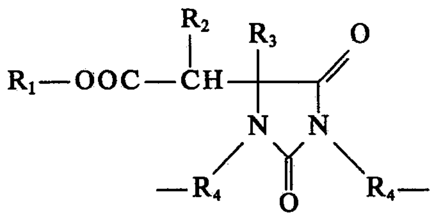

in welcher

$R^1$ einen aliphatischen Rest mit 1—22 C-Atomen, einen aliphatisch-aromatischen Rest mit 7—20 C-Atomen, einen aromatischen Rest mit 6—16 C-Atomen oder einen heterocyclischen Rest mit 3—10 Ring-C-Atomen und 1—3 N-, S- und/oder O-Atomen im Ring bedeutet,
$R^2$ und $R^3$ für Wasserstoff oder Methyl steht und
$R^4$ für einen gegebenenfalls mit Halogen, Alkyl- und/oder Arylgruppen substituierten aliphatischen Rest mit 1—20 C-Atomen, einen cycloaliphatischen Rest mit 5—12 C-Atomen, einen aromatischen Rest mit 6—12 C-Atomen und einen aliphatisch-aromatischen Rest mit 7—20 C-Atomen steht,

dadurch gekennzeichnet, daß ein Fumarsäuremonoester der Formel

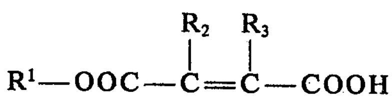

in welcher

$R^1$, $R^2$ und $R^3$ die oben angegebene Bedeutung haben,

mit Mono- bzw. Polyisocyanaten der Formel

$R^4-(NCO)_Z$,

in welcher

$R^4$ die oben angegebene Bedeutung hat und
Z für eine ganze Zahl 1 bis 4 steht,

bei einer Temperatur von 0—450° C umgesetzt wird.

2. Verfahren nach Anspruch 1, dadurch gekennzeichnet, daß in der allgemeinen Formel $R_1$ einen aliphatischen Rest mit 1—6 C-Atomen, einen aromatischen Rest mit 6—10 C-Atomen oder einen aliphatisch-aromatischen Rest mit 7—10 C-Atomen und $R_2$ und $R_3$ Wasserstoff bedeuten.

3. Verfahren nach den Ansprüchen 1—2, dadurch gekennzeichnet, daß als Fumarsäureester die $C_1-C_6$-Monoalkylester verwendet werden.

4. Verfahren nach den Ansprüchen 1—3, dadurch gekennzeichnet, daß als Isocyanate Mono- oder Diisocyanate eingesetzt werden.

**Claims**

1. Process for the preparation of hydantoins of the formula

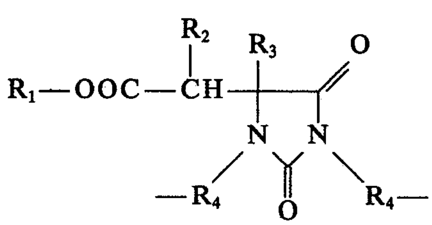

in which

$R^1$ denotes an aliphatic radical with 1—22 C atoms, an aliphatic-aromatic radical with 7—20 C atoms, an aromatic radical with 6—16 C atoms or a heterocyclic radical with 3—10 ring C atoms and 1—3 N, S and/or O atoms in the ring,

$R^2$ and $R^3$ represent hydrogen or methyl, and

$R^4$ represents an aliphatic radical which has 1—20 C atoms and is optionally substituted by halogen, alkyl and/or aryl groups, a cycloaliphatic radical with 5—12 C atoms, an aromatic radical with 6—12 C atoms and an aliphatic-aromatic radical with 7—20 C atoms,

characterised in that a fumaric acid monoester of the formula

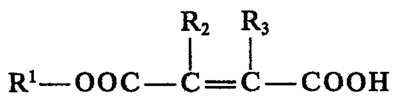

in which

$R^1$, $R^2$ and $R^3$ have the abovementioned meaning,

is reacted with mono- or poly-isocyanates of the formula

$R^4—(NCO)_Z$,

in which

$R^4$ has the abovementioned meaning and

Z represents an integer from 1 to 4,

at a temperature of 0—450° C.

2. Process according to Claim 1, characterised in that in the general formula

$R_1$ denotes an aliphatic radical with 1—6 C atoms, an aromatic radical with 6—10 C atoms or an aliphatic-aromatic radical with 7—10 C atoms and

$R_2$ and $R_3$ denote hydrogen.

3. Process according to Claims 1—2, characterised in that the fumaric acid esters used are the $C_1—C_6$-monoalkyl esters.

4. Process according to Claims 1—3, characterised in that the isocyanates used are mono- or di-isocyanates.

**Revendications**

1. Procédé de production d'hydantoïnes de formule

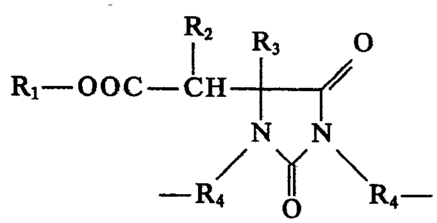

dans laquelle

$R^1$ est un reste aliphatique ayant 1 à 22 atomes de carbone, un reste aliphatique-aromatique ayant 7 à 20 atomes de carbone, un reste aromatique ayant 6 à 16 atomes de carbone ou un reste hétérocyclique ayant 3 à 10 atomes de carbone et 1 à 3 atomes d'azote, de soufre et/ou d'oxygène dans le noyau,

$R^2$ et $R^3$ représentent l'hydrogène ou le groupe méthyle, et

$R^4$ est un reste aliphatique éventuellement substitué avec un halogène, des groupes alkyle et/ou des groupes aryle et ayant 1 à 20 atomes de carbone, un reste cycloaliphatique ayant 5 à 12 atomes de carbone, un reste aromatique ayant 6 à 12 atomes de carbone et un reste aliphatique-aromatique ayant 7 à 20 atomes de carbone,

caractérisé en ce qu'on fait réagir un monoester d'acide fumarique de formule

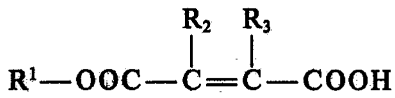

dans laquelle

$R^1$, $R^2$ et $R^3$ ont la définition indiquée ci-dessus, avec des mono- ou des polyisocyanates de formule

$R^4-(NCO)_Z$,

dans laquelle

$R^4$ a la définition indiquée ci-dessus et
Z est un nombre entier de 1 à 4,

à une température de 0 à 450° C.

2. Procédé suivant la revendication 1, caractérisé en ce que dans la formule générale, $R_1$ désigne un reste aliphatique ayant 1 à 6 atomes de carbone, un reste aromatique ayant 6 à 10 atomes de carbone ou un reste aliphatique-aromatique ayant 7 à 10 atomes de carbone et $R_2$ et $R_3$ sont de l'hydrogène.

3. Procédé suivant les revendications 1—2, caractérisé en ce qu'on utilise comme esters d'acide fumarique les esters monoalkyliques en $C_1$ à $C_6$.

4. Procédé suivant les revendications 1—3, caractérisé en ce qu'on utilise comme isocyanates des mono-isocyanates ou des diisocyanates.